# EUROPÄISCHE PATENTSCHRIFT

(11) **EP 3 669 833 B1**
(45) Veröffentlichungstag und Bekanntmachung des Hinweises auf die Patenterteilung: **01.09.2021**
(21) Anmeldenummer: 19213999.6
(22) Anmeldetag: 05.12.2019
(51) Int. Cl.: A61F 5/01

(54) **DYNAMISCHES ORTHESENGELENK**
DYNAMIC ORTHESE JOINT
ARTICULATION DYNAMIQUE D'ORTHÈSE

(30) Priorität: 19.12.2018 DE 102018132887
(43) Veröffentlichungstag der Anmeldung: 24.06.2020
(73) Patentinhaber: Albrecht GmbH, 83071 Stephanskirchen (DE)
(72) Erfinder: OPAHLE, Hans-Georg, 83024 Rosenheim (DE); GRÜTTNER, Bastian, 83627 Warngau / Wall (DE)
(74) Vertreter: Flach Bauer Stahl Patentanwälte Partnerschaft mbB

(56) Entgegenhaltungen:
- WO-A1-95/25489
- US-A- 5 658 241
- US-A1- 2014 308 065

## Beschreibung

Die Erfindung betrifft ein dynamisches Orthesengelenk gemäß dem Oberbegriff des Anspruches 1.

Insbesondere Gelenkkapseln und/oder Bindegewebe weisen beispielsweise nach Bänderoperationen, Unfällen, Entzündungen etc. häufig ein Streck- oder Beugedefizit auf. Dies bedeutet, dass ein distales Körperglied, beispielsweise ein Unterarm oder Unterschenkel, nicht mehr vollständig in seine normale Extensions- oder Flexionslage bezüglich eines proximalen Körperglieds, beispielsweise eines Oberarms oder Oberschenkels, gebracht werden kann.

Um einem derartigen Streck- oder Beugedefizit entgegenzuwirken, werden in bekannter Weise dynamische Orthesen eingesetzt. Derartige Orthesen weisen üblicherweise einen ersten und zweiten Schienenarm auf, die an Körpergliedern befestigbar und über einen Gelenkabschnitt gelenkig miteinander verbunden sind. Im Bereich des Gelenkabschnitts ist eine Krafterzeugungseinrichtung in der Form einer Feder angeordnet, welche die Schienenarme entweder in Extensionsrichtung oder in Flexionsrichtung drängt. Auf die Schienenarme wird somit mittels der Feder eine Vorspannkraft in Extensions- oder Flexionsrichtung aufgebracht, welche dem Streck- oder Beugedefizit entgegenwirkt. Derartige Orthesen werden auch als Quengelschienen oder Redressionsorthesen bezeichnet.

Nachteilig ist bei diesen bekannten Orthesen, dass sie nur in Extensions- oder Flexionsrichtung wirken und nicht für den Einsatz in beiden Richtungen geeignet sind. Liegt sowohl ein Streck- als auch Beugedefizit vor, beispielsweise in Folge von Spastiken, müssen daher üblicherweise die Orthesen gewechselt werden. Dies ist für einen Patienten umständlich und mit einem erhöhten Arbeits- und Zeitaufwand verbunden. Weiterhin erhöhen sich die Behandlungskosten, da sowohl eine in Extensionsrichtung wirkende Orthese als auch eine in Flexionsrichtung wirkende Orthese zur Verfügung gestellt werden müssen.

Aus der US 5658241 A ist ein Orthesengelenk gemäß dem Oberbegriff des Anspruchs 1 bekannt, die diese Nachteile dadurch zu vermeiden versucht, dass das Orthesengelenk auf einfache Weise demontierbar ausgebildet ist, wodurch die Möglichkeit geschaffen wird, die Feder in unterschiedlichen Lagen im Orthesengelenk festzulegen und die Richtung der Federkraft umzukehren.

Weitere Orthesengelenke, bei denen die Feder in unterschiedlichen Ausrichtungen montiert werden kann, nachdem das Orthesengelenk auseinandergebaut wurde, sind aus der WO 95/25489 A1 und US 2014/0308065 A1 bekannt.

Der Erfindung liegt die Aufgabe zugrunde, ein dynamisches Orthesengelenk der eingangs genannten Art zu schaffen, das sowohl für die Behandlung von Beugedefiziten als auch zur Behandlung von Streckdefiziten von Körpergliedern geeignet ist und dabei eine besonders einfache Handhabung ermöglicht.

Diese Aufgabe wird erfindungsgemäß durch ein dynamisches Orthesengelenk mit den Merkmalen des Anspruches 1 gelöst. Vorteilhafte Ausführungsformen der Erfindung sind in den weiteren Ansprüchen beschrieben.

Beim erfindungsgemäßen Orthesengelenk ist das Gehäuse relativ zu beiden Schienenarmen drehbar gelagert. Die mit dem zweiten Federende in Wirkverbindung stehende Federkraftübertragungseinrichtung weist eine Kupplungsscheibe auf, die um eine Schwenkachse drehbar ist. Das Orthesengelenk weist einen Umschaltmechanismus zum Umschalten zwischen einem ersten Betriebszustand, in dem die Federkraft die Schienenarme in Extensionsrichtung drängt, und einem zweiten Betriebszustand auf, in dem die Federkraft die Schienenarme in Flexionsrichtung drängt, wobei der Umschaltmechanismus eine zwischen den Schienenarmen, dem Gehäuse und der Federkraftübertragungseinrichtung wirkende lösbare Drehkopplungseinrichtung umfasst, mit welcher der erste und zweite Schienenarm wahlweise mit dem Gehäuse oder der Federkraftübertragungseinrichtung in Drehkopplungsverbindung bringbar sind. Das Gehäuse ist bei gelöster Drehkopplungseinrichtung zwischen einer ersten Drehposition, in der das Gehäuse mit dem ersten Schienenarm drehfest verbindbar ist, während die Kupplungsscheibe mit dem zweiten Schienenarm drehfest verbindbar ist, und einer zweiten Drehposition drehbar, in der das Gehäuse mit dem zweiten Schienenarm drehfest verbindbar ist, während die Kupplungsscheibe mit dem ersten Schienenarm drehfest verbindbar ist.

Das erfindungsgemäße Orthesengelenk bietet den Vorteil, dass das Orthesengelenk auf einfache und schnelle Weise von einem in Extensionsrichtung wirkenden Orthesengelenk in ein in Flexionsrichtung wirkendes Orthesengelenk, und umgekehrt, umgeschaltet werden kann. Das Umschalten erfolgt dadurch, dass diejenige Federkraft, die ursprünglich auf den ersten Schienenarm einwirkt, auf den zweiten Schienenarm umgeleitet wird, während diejenige Federkraft, die ursprünglich auf den zweiten Schienenarm einwirkt, auf den ersten Schienenarm umgeleitet wird. Vorzugsweise ist dieses Umschalten werkzeuglos und ohne Abnehmen des Orthesengelenks von den Körpergliedern möglich. Streck- und Beugedefizite eines Patienten können daher ohne umständliches Austauschen des Orthesengelenks behandelt werden. Da hierzu nicht zwei unterschiedliche Orthesengelenke, sondern nur ein einziges Orthesengelenk erforderlich ist, können auch die Behandlungskosten gesenkt werden. Der Wechsel von einem in Extensionsrichtung wirkenden Orthesengelenk in ein in Flexionsrichtung wirkendes Orthesengelenk, und umgekehrt, ist auf besonders einfache Weise möglich, da lediglich das Gehäuse um einen bestimmten Winkelbetrag gedreht werden muss und die Schienenarme etwas geschwenkt werden müssen, um das Orthesengelenk umzuschalten.

Der erste oder zweite Schienenarm kann direkt mit der Kupplungsscheibe zusammenwirken, die mit dem zweiten Federende verbunden ist, wodurch eine sehr platzsparende Anordnung mit geringer Baugröße geschaffen wird. Es ist jedoch auch denkbar, die Schienenarme über andere oder zusätzliche Zwischenglieder, die Teil der Federkraftübertragungseinrichtung sind, mit dem zweiten Federende zu verbinden.

Vorteilhafterweise umfasst die Drehkopplungseinrichtung Rastausnehmungen, die am Gehäuse und an der Kupplungsscheibe angeordnet sind, und Sperrriegel, die an den Schienenarmen festgelegt und zwischen einer in die Rastausnehmungen eingreifenden Drehblockierstellung und einer aus den Rastausnehmungen entfernten Drehfreigabestellung bewegbar sind. Alternativ hierzu ist es auch möglich, die Rastausnehmungen nach Art einer kinematischen Umkehr an den Schienenarmen vorzusehen, während die Sperrriegel am Gehäuse und an der Kupplungsscheibe festgelegt sind.

Vorteilhafterweise bestehen die Sperrriegel aus Schiebe- oder Schwenkriegeln, die verschiebbar oder schwenkbar an den Schienenarmen befestigt sind. Auch hier ist es möglich, dass die Schiebe- oder Schwenkriegel nach Art einer kinematischen Umkehr nicht an den Schienenarmen, sondern am Gehäuse und an der Kupplungsscheibe befestigt sind.

Vorteilhafterweise weist das Gehäuse zwei Rastausnehmungen auf, die in Umfangsrichtung des Gehäuses um 100° bis 150°, vorzugsweise 120° bis 130°, zueinander beabstandet angeordnet sind. Hierdurch ist es möglich, die Umstellung des Orthesengelenks von Extension auf Flexion, und umgekehrt, durchzuführen, ohne dass das Gehäuse genau um denjenigen Winkelbetrag relativ zu den Schienenarmen gedreht werden muss, den die beiden Schienenarme zueinander einnehmen. Hierdurch ergibt sich eine größere Variabilität bei der Gestaltung des Orthesengelenks.

Vorteilhafterweise weist das Gehäuse ein napfförmiges Gehäusehauptteil und ein axial über das Gehäusehauptteil vorragendes, sich lediglich über einen Teil von dessen Umfang erstreckendes Gehäusesegment auf, in dem mindestens eine der Rastausnehmungen vorgesehen ist. Dadurch, dass sich dieses Gehäusesegment nur über einen Teil des Umfangs, beispielsweise 170° bis 190°, insbesondere 180°, erstreckt, existiert im übrigen Umfangsbereich ein genügend großer Freiraum, in dem sich die Sperrriegel beim Verschwenken der Schienenarme bewegen können. Weiterhin können die Enden des vorragenden Gehäusesegments als Schwenkanschläge für die Schienenarme und/oder für die Kupplungsscheibe benutzt werden.

Vorzugsweise ist mindestens einer der Sperrriegel mittels einer Feder in die Drehblockierstellung vorgespannt. Hierdurch kann auf einfache Weise verhindert werden, dass sich die Drehblockierung zwischen Schienenarm und Gehäuse bzw. zwischen Schienenarm und Federkraftübertragungseinrichtung unabsichtlich löst. Alternativ oder zusätzlich ist es jedoch auch möglich, dass mindestens einer der Sperrriegel einen in der Drehblockierstellung selbsthemmenden Verriegelungskopf mit einer schräg zur Verschiebe- oder Schwenkrichtung der Sperrriegel angeordneten und mit einer Wand der Rastausnehmungen in Anlage bringbaren Anlagefläche aufweist, welche die Wand hintergreift. In diesem Fall existiert bei eingerastetem Sperrriegel eine Formschlussverbindung zwischen Sperrriegel und Wand der Rastausnehmung, welche das unerwünschte Entfernen des Sperrriegels aus der Rastausnehmung verhindert. Ein Entfernen des Sperrriegels ist dann dadurch möglich, dass dasjenige Element, das die Rastausnehmung aufweist, geringfügig manuell gedreht wird, wodurch der Sperrriegel freigegeben wird.

Gemäß einer besonders vorteilhaften Ausführungsform weist das Orthesengelenk eine Federkraftblockiereinrichtung zum Abkoppeln der Federkraft von den Schienenarmen auf. Hierdurch ist es möglich, die Umstellung des Orthesengelenks von Extension auf Flexion, und umgekehrt, losgelöst von der Vorspannkraft der Feder durchzuführen. Hierdurch ist eine besonders einfache und sichere Umstellung möglich.

Vorteilhafterweise ist die Federkraftblockiereinrichtung ausgebildet, mit der Kupplungsscheibe der Federkraftübertragungseinrichtung derart zusammenzuwirken, dass die Drehung der Kupplungsscheibe in Richtung der Federkraft wahlweise blockiert oder freigegeben wird. Hierdurch kann die Federkraftblockiereinrichtung auf besonders einfache und platzsparende Weise gestaltet werden.

Vorteilhafterweise weist die Federkraftblockiereinrichtung einen an der Kupplungsscheibe angeordneten Anschlag und eine Sperrklinke auf, die mittels einer Handhabe in die Bewegungsbahn des Anschlags hineinbewegbar und aus dieser Bewegungsbahn entfernbar ist. Hierdurch kann die Federkraftblockiereinrichtung auf besonders einfache Weise realisiert werden.

Die Erfindung wird nachfolgend anhand der Zeichnungen beispielhaft näher erläutert. Es zeigen:
- Figur 1:: eine räumliche Ansicht des erfindungsgemäßen Orthesengelenks von einer ersten Seite her ohne Befestigungsmittel zur Befestigung an Körpergliedern;
- Figur 2:: eine räumliche Ansicht auf das Orthesengelenk von Figur 1 schräg von unten mit Blick auf dessen zweite Seite;
- Figur 3:: eine räumliche Ansicht des Orthesengelenks mit Blick auf dessen zweite Seite unter einem anderen Blickwinkel;
- Figur 4:: einen Schnitt durch den Gelenkabschnitt in einer Ebene, die zwischen einer Gehäuseabdeckscheibe und einer Kupplungsscheibe liegt;
- Figur 5:: das Orthesengelenk in Explosionsdarstellung;
- Figur 6:: eine Darstellung eines zentralen Schraub-elements;
- Figur 7:: eine Darstellung einer Kupplungsscheibe;
- Figur 8:: eine Darstellung einer Feder;
- Figur 9:: eine Darstellung eines Federträgers;
- Figur 10:: eine Darstellung eines Verstellzahnrads;
- Figur 11:: eine Darstellung eines Gehäusesegments des Gehäuses;
- Figur 12:: eine Darstellung des Gehäuses; und
- Figuren 13a bis 13g:: verkürzt dargestellte, schematische Darstellungen des Orthesengelenks in verschiedenen Stellungen zur Verdeutlichung der Funktionsweise des Umschaltmechanismus.

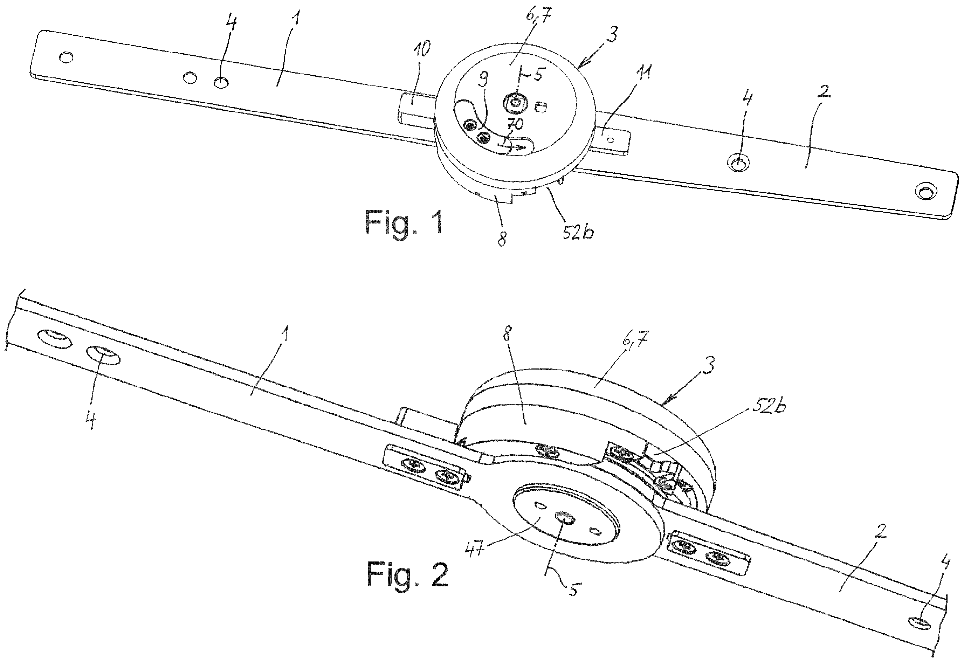

Figur 1 zeigt ein dynamisches Orthesengelenk mit einem ersten Schienenarm 1 und einem zweiten Schienenarm 2, die mittels eines Gelenkabschnitts 3 gelenkig miteinander verbunden sind. An den Schienenarmen 1, 2 sind Befestigungsbohrungen 4 vorgesehen, um beispielsweise Schrauben oder Nieten zum Befestigen von nicht dargestellten Befestigungselementen, insbesondere Klettbänder, Schalen oder dergleichen, hindurchführen zu können. Mit derartigen Befestigungselementen können die Schienenarme 1, 2 in bekannter Weise an mittels eines Körpergelenks verbundenen Gliedmaßen, beispielsweise am Oberund Unterschenkel, Ober- und Unterarm etc., befestigt werden. Das erfindungsgemäße Orthesengelenk kann insbesondere für das Knie-, Ellbogen-, Hand-, Sprung- und Hüftgelenk verwendet werden. Darüber hinaus ist es auch möglich, das erfindungsgemäße Orthesengelenk an einer anderen orthopädischen Vorrichtung, insbesondere einem Fixateur externe, zu befestigen.

Die beiden Schienenarme 1, 2 sind im Gelenkabschnitt 3 derart gelenkig miteinander verbunden, dass sie um eine gemeinsame Schwenkachse 5 geschwenkt werden können.

Der Gelenkabschnitt 3 umfasst ein Gehäuse 6, das im gezeigten Ausführungsbeispiel aus einem napf- oder tellerförmigen Gehäusehauptteil 7 und einem zusätzlichen Gehäusesegment 8 besteht, das sich in axialer Richtung des Gelenkabschnitts 3 über das Gehäusehauptteil 7 hinaus in medialer Richtung erstreckt, während es sich in Umfangsrichtung lediglich über einen Teil des Umfangs des Gehäuses 6, zweckmäßigerweise über etwa 180° erstreckt.

Das Gehäuse 6 dient zur Aufnahme eines nachfolgend noch näher erläuterten Federmechanismus sowie einer Federkraftverstelleinrichtung und einer Federkraftblockiereinrichtung. Es handelt sich beim dargestellten Orthesengelenk somit um eine sog. Quengelschiene oder Redressionsorthese zum Aufbringen einer Vorspannkraft in Flexions- oder Extensionsrichtung zwischen dem ersten Schienenarm 1 und dem zweiten Schienenarm 2, wobei die vorzugsweise vorhandenen Befestigungsmittel zum Befestigen des Orthesengelenks an Körpergliedern nicht dargestellt sind.

Aus Figur 1 ist weiterhin eine Handhabe 9 in der Form eines Schiebers ersichtlich, der, wie nachfolgend noch näher erläutert wird, zum Abkoppeln einer Federkraft von den Schienenarmen 1, 2 dient.

Ein erster Sperrriegel 10, der verschiebbar am ersten Schienenarm 1 befestigt ist, und ein zweiter Sperrriegel 11, der verschiebbar am zweiten Schienenarm 2 befestigt ist, sind Teil eines Umschaltmechanismus, mit dem das Orthesengelenk von einem ersten Betriebszustand, in dem die Federkraft die Schienenarme 1, 2 in Extensionsrichtung drängt, in einen zweiten Betriebszustand umgeschaltet werden kann, in dem die Federkraft die Schienenarme 1, 2 in Flexionsrichtung drängt. Dies wird, wie später noch näher erläutert wird, dadurch bewirkt, dass die Schienenarme 1, 2 mittels der Sperrriegel 10, 11 wahlweise mit dem Gehäuse 6 oder mit einer relativ zum Gehäuse 6 drehbaren Federkraftübertragungseinrichtung 34, insbesondere einer Kupplungsscheibe 34a, drehgekoppelt werden können.

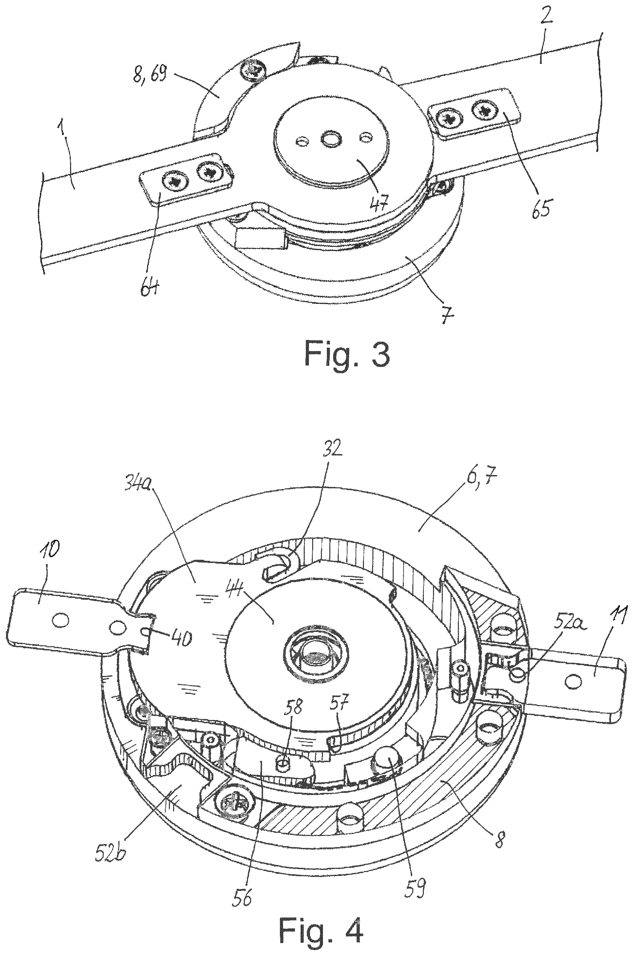

Im Folgenden wird anhand der Figuren 2 bis 12 der Aufbau des Orthesengelenks näher beschrieben.

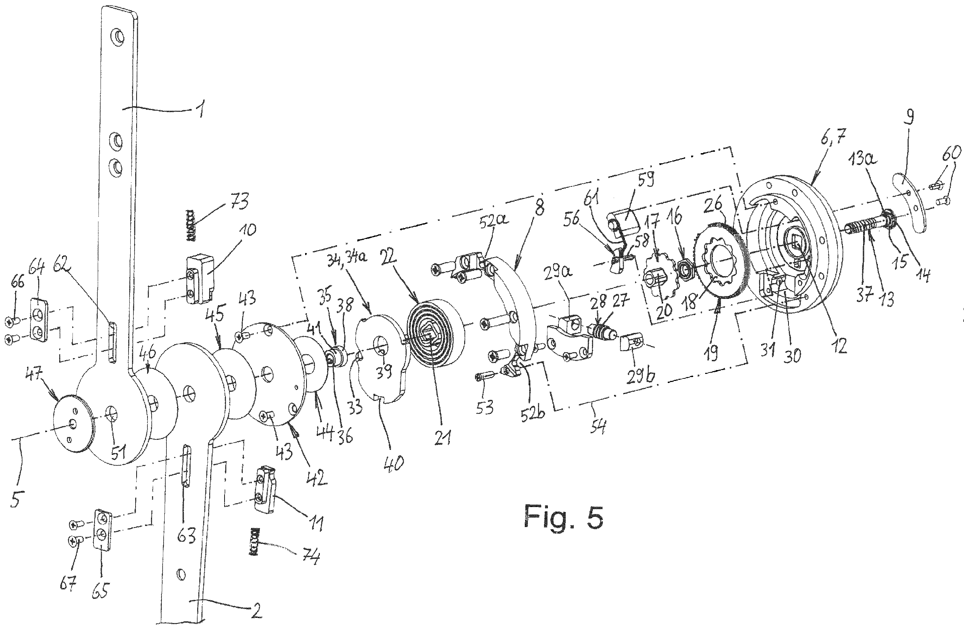

Wie aus Figur 5 ersichtlich, weist das Gehäuse 6 eine mittige Durchgangsöffnung 12 auf, durch die ein bolzenförmiges Achselement 13 hindurchgeführt werden kann. Hierbei kann das Achselement 13 bis zu seinem Kopf 14 durch das Gehäuse 6 hindurchgesteckt werden. Ein benachbart zum Kopf 14 angeordneter Vierkant 15, der an die Vierkantform der Durchgangsöffnung 12 abgestimmt ist, verhindert ein Drehen des Achselements 13 relativ zum Gehäuse 6. Mittels einer Mutter 16, die von der gegenüberliegenden Seite des Gehäuses 6 her auf ein Außengewinde 13a des Achselements 13 aufgeschraubt wird, wird das Achselement 13 am Gehäuse 6 festgelegt. Die Längsachse des Achselements 13 bildet die Schwenkachse 5 des Orthesengelenks.

Auf dem Achselement 13 ist ein Federträger 17 drehbar gelagert, der in eine Vertiefung 18 eines Verstellzahnrads 19 eingesetzt und drehfest mit diesem gekoppelt ist.

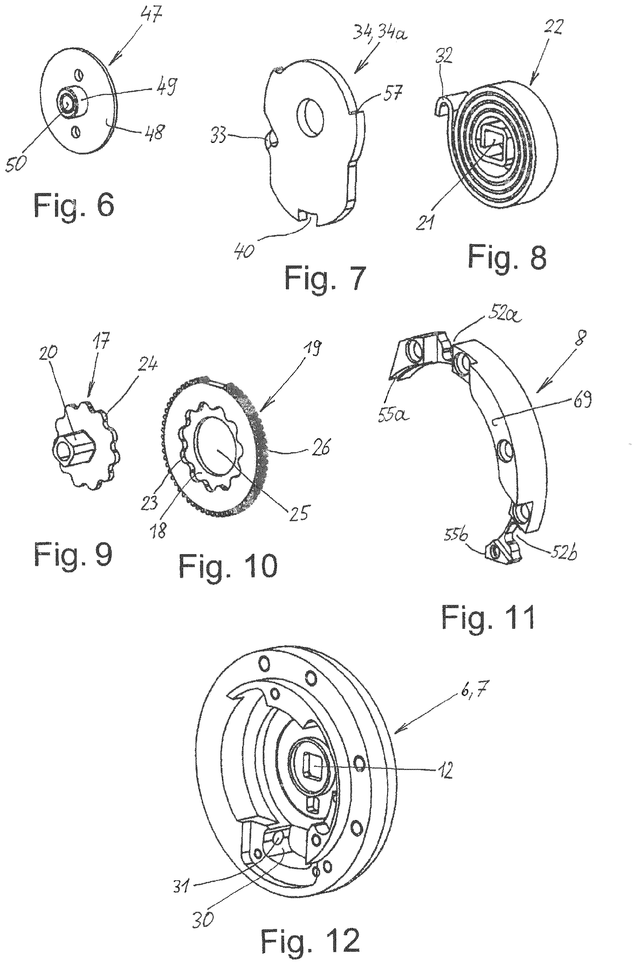

Wie aus Figur 9 ersichtlich, weist der Federträger 17 einen Federeingriffabsatz 20 mit einer Außenkontur auf, die an eine Innenkontur eines ersten, radial inneren Federendes 21 einer Feder 22 (Figur 8) angepasst ist. Das erste Federende 21 kann dadurch auf den Federeingriffabsatz 20 des Federträgers 17 drehfest aufgesteckt werden. Die drehfeste Verbindung erfolgt durch eine mehreckige, im dargestellten Ausführungsbeispiel viereckige, Formgebung des Federeingriffabsatzes 20 und des ersten Federendes 21.

Der Federträger 17 dient dazu, das erste Federende 21 stationär relativ zum Gehäuse 6 zu halten. Weiterhin dient der Federträger 17 zusammen mit dem drehfest mit dem Federträger 17 koppelbaren Verstellzahnrad 19 zum Einstellen der Vorspannkraft der Feder 22.

Zur drehfesten Verbindung des Federträgers 17 mit dem Verstellzahnrad 19 weist, wie aus Figur 9 und 10 ersichtlich, die Vertiefung 18 des Verstellzahnrads 19 einen wellenförmigen Umfangsrand 23 auf, während der Federträger 17 eine daran angepasste, entsprechend wellenförmige Außenkontur 24 aufweist. Andere lösbare formschlüssige Verbindungen sind ebenfalls möglich. Diese formschlüssige Verbindung dient zur Grobeinstellung der Drehposition des Federträgers 17 relativ zum Verstellzahnrad 19 in Abhängigkeit der jeweiligen Feder 22.

Eine mittige Durchgangsöffnung 25 des Verstellzahnrads 19 hat einen größeren Durchmesser als die Mutter 16, so dass das Verstellzahnrad 19 zusammen mit dem Federträger 17 um die Mutter 16 herumgedreht werden kann.

Das Verstellzahnrad 19 weist weiterhin eine Stirnverzahnung 26 auf, die mit einem Schneckengewinde 27 einer Schnecke 28 (Figuren 5 und 12) in Eingriff ist. Die Schnecke 28 ist mit zylindrischen Endabschnitten in Lagerelementen 29a, 29b drehbar gelagert. Die Lagerelemente 29a, 29b sind in eine randseitige, taschenartige Vertiefung 30 des Gehäuses 6 (Figur 12) derart eingesetzt, dass die Längs- bzw. Drehachse der Schnecke 28 quer zur Schwenkachse 5 verläuft.

Das Schneckengewinde 27 ist selbsthemmend. Die durch die Schnecke 28 eingestellte Drehposition des Verstellzahnrads 19 und damit des Federträgers 17 relativ zum Gehäuse 6 ist damit festgelegt und kann sich ohne manuellen Eingriff nicht ändern. Andererseits kann die Vorspannkraft der Feder 22 erhöht oder verringert werden, indem die Drehposition des Verstellzahnrads 19 und damit des Federträgers 17 und des ersten Federendes 21 durch manuelles Drehen der Schnecke 28 verändert wird. Im gezeigten Ausführungsbeispiel kann die Schnecke 28 mittels eines nicht dargestellten Inbusschlüssels gedreht werden, der durch ein seitliches Loch 31 des Gehäuses 6 sowie durch die Lagerbohrung des Lagerelements 29a hindurch in eine axiale Inbusvertiefung der Schnecke 28 eingeführt werden kann.

Bei der Feder 22 handelt es sich, wie aus den Figuren 5 und 8 ersichtlich, um eine Spiralfeder mit um die Schwenkachse 5 herumlaufenden Federwindungen. Die Feder 22 weist ein zweites, radial äußeres Federende 32 auf, das mit einem exzentrisch angeordneten Mitnehmer 33 einer Kupplungsscheibe 34a (Figuren 5 und 7) verbunden ist. Im gezeigten Ausführungsbeispiel handelt es sich beim Mitnehmer 33 um eine im Randbereich der Kupplungsscheibe 34a angeordnete Lasche, die über die Hauptebene der Kupplungsscheibe 34a parallel zur Schwenkachse 5 in Richtung der Feder 22 vorsteht und vom zweiten Federende 32, das U-förmig zurückgebogen ist, übergriffen wird. Andere Mitnehmer bzw. Drehkopplungselemente zwischen Kupplungsscheibe 34a und dem Federende 32 sind ohne weiteres möglich. Maßgebend ist, dass für die Feder 22 eine Drehkraft auf die Kupplungsscheibe 34a ausgeübt werden kann, welche diese um die Schwenkachse 5 zu drehen versucht.

Die Kupplungsscheibe 34a ist auf einem in Figur 5 dargestellten hülsenartigen Lagerelement 35 drehbar gelagert. Das Lagerelement 35 weist eine axial durchgehende Gewindebohrung 36 auf, mit der das Lagerelement 35 auf einen Gewindeabschnitt 37 des Achselements 13 aufgeschraubt werden kann, bis das Lagerelement 35 an der Stirnseite des Federeingriffabsatzes 20 des Federträgers 17 anliegt. Ein zylinderförmiger Lagerabschnitt 38 des Lagerelements 35 erstreckt sich dabei in eine Lagerbohrung 39 der Kupplungsscheibe 34a hinein.

Die Kupplungsscheibe 34a weist ferner in ihrem Randbereich eine randseitig offene Rastausnehmung 40 auf. Diese Aussparung 40 ist derart angeordnet und ausgebildet, dass die beiden Sperrriegel 10, 11, die am ersten Schienenarm 1 und am zweiten Schienenarm 2 befestigt sind, in ihrer Verriegelungsstellung in diese Rastausnehmung 40 eingreifen können. Je nachdem, ob der erste Sperrriegel 10 oder der zweite Sperrriegel 11 in die Rastausnehmung 40 eingreift, ist somit entweder der erste Schienenarm 1 oder der zweite Schienenarm 2 mit der Kupplungsscheibe 34a drehgekoppelt. Der jeweils andere Schienenarm 1, 2 kann dann über den jeweils anderen Sperrriegel 10, 11 mit dem Gehäusesegment 8 und damit mit dem Gehäuse 6 und dem inneren Federende 21 drehgekoppelt werden.

Wie weiterhin aus Figur 5 ersichtlich, weist das Lagerelement 35 einen weiteren zylinderförmigen Lagerabschnitt 41 auf, auf den eine Gehäuseabdeckscheibe 42 aufgesetzt werden kann. Die Gehäuseabdeckscheibe 42 wird mit zwei Schrauben 43 am Gehäusehauptteil 7 festgeschraubt.

Um die Reibung zwischen der Kupplungsscheibe 34a und der Gehäuseabdeckscheibe 42 zu verringern, ist dazwischen eine Reibungsverminderungsscheibe 44, beispielsweise aus Teflon, angeordnet.

Der Lagerabschnitt 41 des Lagerelements 35 erstreckt sich durch eine mittige Bohrung in der Gehäuseabdeckscheibe 42 hindurch und darüber hinaus, wobei auch eine weitere Reibungsverminderungsscheibe 45 und ein Endbereich des zweiten Schienenarms 2 auf diesem Lagerabschnitt 41 drehgelagert sind.

Wie aus Figur 5 weiterhin ersichtlich, sind die beiden Schienenarme 1, 2 benachbart zueinander angeordnet. Zwischen dem ersten Schienenarm 1 und dem zweiten Schienenarm 2 ist lediglich eine weitere Reibungsverminderungsscheibe 36 angeordnet, die zur Reibungsverminderung dient, wenn die beiden Schienenarme 1, 2 relativ zueinander um die Schwenkachse 5 verschwenkt werden.

In den Figuren 5 und 6 ist ein Schraubelement 47 dargestellt, welches das axial äußerste Element des Gelenkabschnitts 3 darstellt und auf den Gewindeabschnitt 37 des Achselements 13 aufgeschraubt werden kann. Das Schraubelement 47 weist einen scheibenförmigen Kopfabschnitt 48 und einen zylinderförmigen Lagerabschnitt 49 auf, der mittig und in axialer Richtung über den Kopfabschnitt 48 vorsteht und eine Gewindebohrung 50 aufweist. Mittels dieser Gewindebohrung 50 ist das Schraubelement 47 auf das Achselement 13 aufschraubbar, bis die Stirnseite des Lagerabschnitts 49 an der Stirnseite des Lagerelements 35 anschlägt. Der Lagerabschnitt 49 des Schraubelements 47 erstreckt sich dabei durch eine Lagerbohrung 51 in einem Endbereich des ersten Schienenarms 1 hindurch, so dass sich der erste Schienenarm 1 um den Lagerabschnitt 49 herumdrehen kann.

Wie bereits ausgeführt, kann jeder der beiden Schienenarme 1, 2 mittels der Sperrriegel 10, 11 mit dem Gehäuse 6 drehgekoppelt werden. Dies erfolgt dadurch, dass die Sperrriegel 10, 11 in und außer Verriegelungseingriff mit Rastausnehmungen 52a, 52b des Gehäusesegments 8 (Figuren 5 und 11) gebracht werden können. Diese Rastausnehmungen 52a, 52b sind in gleicher Weise wie die Rastausnehmung 40 der Kupplungsscheibe 34a radial nach außen offen und derart ausgebildet und angeordnet, dass die Sperrriegel 10, 11 in die Rastausnehmungen 52a, 52b eingreifen können. Weiterhin sind die Rastausnehmungen 52a, 52b in Umfangsrichtung des Gehäuses 7 bzw. des Gehäusesegments 8 gesehen um etwa 130° zueinander versetzt angeordnet. Das Vorhandensein von zwei Rastausnehmungen 52a, 52b am Gehäusesegment 8 bewirkt, dass dann, wenn sich die beiden Schienenarme 1, 2 beim Umschalten von Extensions- auf Flexionsbetrieb (oder umgekehrt) in einer Stellung von etwa 90° zueinander befinden, das Gehäuse 6 zusammen mit dem Gehäusesegment 8 lediglich um einen Winkel von etwa 90° um die Schwenkachse 5 herumgedreht werden muss, um die Verriegelung zwischen den Schienenarmen 1, 2 und dem Gehäuse 6 zu wechseln. Von den beiden Rastausnehmungen 52a, 52b wirkt immer nur eine Rastausnehmung 52a, 52b mit den Schienenarmen 1,2 zusammen, während die andere Rastausnehmung 52a, 52b frei bleibt.

Das Gehäusesegment 8 besteht aus einem kreisbogenförmigen Randsegment, das im gezeigten Ausführungsbeispiel mit fünf Schrauben 53 in einem randseitigen Bereich des Gehäusehauptteils 7 festgeschraubt ist. In Figur 5 ist dies mittels der strichpunktierten Linie 54 nur anhand der untersten Schraube 53 angedeutet. Das Gehäusesegment 8 steht in axialer Richtung über das Gehäusehauptteil 7 vor.

Da sich das Gehäusesegment 8 in Umfangsrichtung nur über etwa 180° erstreckt, bleibt zwischen den Enden 55a, 55b des Gehäusesegments 8 (Figur 11) in demjenigen Randbereich des Gehäuses 6, in dem das Gehäusesegment 8 nicht vorhanden ist, ebenfalls ein Freiraum von etwa 180°, in welchem der sich in diesen Freiraum hinein erstreckende Randbereich der Kupplungsscheibe 34a, in welchem sich die Rastausnehmung 40 befindet, verschwenken lässt.

Beim Umschalten des Orthesengelenks vom Extensions- auf Flexionsbetrieb (und umgekehrt) ist es zweckmäßig, wenn die Federkraft von den Schienenarmen 1, 2 abgekoppelt wird, um einerseits ein leichtes Verdrehen des Gehäuses 6 relativ zu den Schienenarmen 1, 2 zu ermöglichen und andererseits unerwartete Schwenkbewegungen zu vermeiden. Hierzu ist ein Federkraftblockiermechanismus vorgesehen, der eine Sperrklinke 56 umfasst, die mit einem randseitigen Anschlag 57 (Figur 7) der Kupplungsscheibe 34a zusammenwirkt. Die Sperrklinke 56 ist mittels eines Lagerstifts 58 (Figur 5) wippenartig schwenkbar im Gehäusehauptteil 7 gelagert, wobei die Schwenkachse der Sperrklinke 56 parallel zur Schwenkachse 5 verläuft. Durch Verschwenken der Sperrklinke 56 kann ein Verriegelungsabschnitt 56a der Sperrklinke 56 in den bzw. aus dem Bewegungsbereich des Anschlags 57 der Kupplungsscheibe 34a gebracht werden.

Das Verschwenken der Sperrklinke erfolgt über einen manuell in Umfangsrichtung des Gehäuses 6 verschiebbaren Sperrschieber 59 (Figur 5), der über zwei Schrauben 60, die sich durch ein bogenförmiges Langloch im Gehäusehauptteil 7 hindurch erstrecken, mit der Handhabe 9 fest verbunden ist. Am Sperrschieber 59 ist ein Federelement 61 befestigt, das von radial außen auf die Sperrklinke 56 drückt und die Sperrklinke 56 je nach Stellung des Sperrschiebers 59 über den Anschlag 57 der Kupplungsscheibe 34a anhebt oder in dessen Bewegungsbahn hineindrückt. Befindet sich die Sperrklinke 56 in ihrer Verriegelungsstellung, kann sich die Kupplungsscheibe 34a nicht mehr in derjenigen Drehrichtung, in der sie durch die Feder 22 gedrängt wird, relativ zum Gehäuse 6 drehen, so dass die Kraftübertragung auf die Schienenarme 1, 2 gesperrt bzw. abgekoppelt ist.

Wie bereits ausgeführt, sind die Sperrriegel 10, 11 verschiebbar am ersten Schienenarm 1 bzw. zweiten Schienenarm 2 festgelegt. Die Verschieberichtung erfolgt in Längsrichtung der Schienenarme 1, 2 entlang von Langlöchern 62, 63. Bei den Sperrriegeln 10, 11 handelt es sich um Schieberiegel, die auf einer Seite der Schienenarme 1 bzw. 2 angeordnet und mittels Gegenplatten 64, 65, die auf der gegenüberliegenden Seite der Schienenarme 1, 2 angeordnet sind, und Schrauben 66, 67, die durch die Langlöcher 62, 63 hindurchgeführt sind, an den Schienenarmen 1, 2 verschiebbar befestigt sind. Werden die Sperrriegel 10, 11 von der Schwenkachse 5 wegbewegt, geraten die Sperrriegel 10, 11 außer Eingriff mit den Rastausnehmungen 40, 52a oder 52b. Werden die Sperrriegel 10, 11 dagegen zur Schwenkachse hin verschoben, können sie in Eingriff mit den Rastausnehmungen 40, 52a oder 52b gebracht werden, wodurch eine Drehverriegelung entweder mit der Kupplungsscheibe 34a oder mit dem Gehäuse 6 geschaffen wird.

Zweckmäßigerweise sind die Sperrriegel 10, 11 mit Federn 73, 74 radial nach innen in ihre Verriegelungsstellung vorgespannt. Im gezeigten Ausführungsbeispiel sind die Federn 73, 74 in die Langlöcher 62, 63 der Schienenarme 1, 2 eingesetzt und stützen sich einerseits jeweils am äußeren Ende des Langlochs 62, 63 und andererseits am Sperrriegel 10 bzw. 11 ab.

Die Sperrriegel 10, 11, mit denen die Schienenarme 1, 2 wahlweise mit der Kupplungsscheibe 34a oder dem Gehäuse 6 drehgekoppelt werden können, sind somit Teil einer lösbaren Drehkopplungeinrichtung, mit welcher die Drehkopplung zwischen den genannten Teilen hergestellt oder gelöst werden kann. Ist beispielsweise der erste Schienenarm 1 mit dem Gehäuse 6 und der zweite Schienenarm 2 mit der Kupplungsscheibe 34a drehgekoppelt und wirkt bei dieser Anordnung die Federkraft in Extensionsrichtung, kann das Orthesengelenk in ein in Flexionsrichtung wirkendes Orthesengelenk dadurch umgeschaltet werden, dass der erste Schienenarm 1 mit der Kupplungsscheibe 34a und der zweite Schienenarm 2 mit dem Gehäuse 6 drehgekoppelt wird. Diese lösbare Drehkopplungseinrichtung bildet somit einen Umschaltmechanismus zum Umschalten zwischen einem ersten Betriebszustand, in dem die Federkraft die Schienenarme 1, 2 in Extensionsrichtung drängt, und einem zweiten Betriebszustand, in dem die Federkraft die Schienenarme 1, 2 in Flexionsrichtung drängt. Ein weiterer zweckmäßiger Bestandteil dieses Umschaltmechanismus ist die Federkraftblockiereinrichtung, die im gezeigten Ausführungsbeispiel die schwenkbar am Gehäuse 6 gelagerte Sperrklinke 56 und den damit zusammenwirkenden Anschlag 57 der Kupplungsscheibe 34a umfasst.

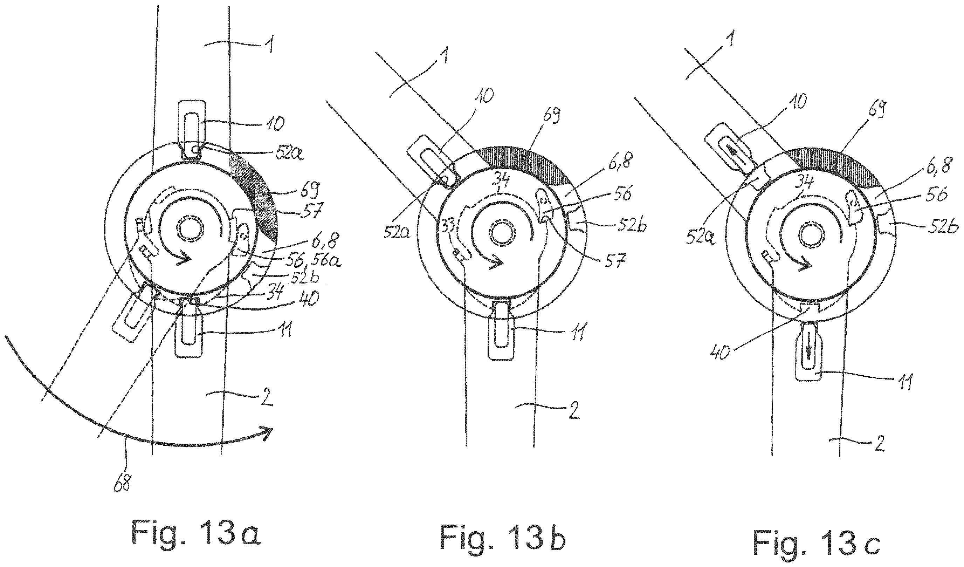

Anhand der Figuren 13a bis 13g wird im Folgenden die Funktionsweise beim Umschalten von einem in Extensionsrichtung wirkenden Orthesengelenk in ein in Flexionsrichtung wirkendes Orthesengelenk näher erläutert. Für ein Umschalten von Flexion auf Extension gilt entsprechendes.

Figur 13a zeigt das Orthesengelenk mit durchgezogenen Linien in ihrer maximalen Extensionsstellung, in welcher die Schienenarme 1, 2 zueinander in einem Winkel von etwa 180° verlaufen. Die Federkraft drängt hierbei den zweiten Schienenarm 2 in Richtung des Pfeils 68 im Gegenuhrzeigersinn, wenn sich der zweite Schienenarm 2 in einer in Figur 13a gestrichelt dargestellten abgewinkelten Zwischenstellung, die kleiner als 180° ist, befindet. Die in den Figuren 13a bis 13g schraffiert dargestellte Fläche entspricht einem erhabenen Abschnitt 69 des Gehäusesegments 8 von Figur 11 und stellt damit eine gehäusefeste Fläche dar.

In Figur 13a greift der erste Sperrriegel 10, der am ersten Schienenarm 1 befestigt ist, in die Rastausnehmung 52a des Gehäusesegments 8 und damit des Gehäuses 6 ein. Der erste Schienenarm 1 ist daher mit dem Gehäuse 6 und - über das Verstellzahnrad 19 und den Federträger 17 - mit dem ersten, inneren Federende 21 drehgekoppelt. Die weitere Rastausnehmung 52b des Gehäusesegments 8 ist unbesetzt.

Der zweite Sperrriegel 11, der am zweiten Schienenarm 2 befestigt ist, greift in die Rastausnehmung 40 der Kupplungsscheibe 34a ein, die in den Figuren 13a bis 13g mit einer gestrichelten Linie dargestellt ist. Der zweite Schienenarm 2 ist daher mit der Kupplungsscheibe 34a verriegelt bzw. drehgekoppelt und über den Mitnehmer 33 der Kupplungsscheibe 34a mit dem zweiten, äußeren Federende 32 in Wirkverbindung.

Die Handhabe 9 und damit der Sperrschieber 59 der Federkraftblockiereinrichtung befindet sich in einer Freigabestellung, in welcher das Federelement 61 (Figur 5) derart auf die Sperrklinke 56 drückt, dass deren Verriegelungsabschnitt 56a radial nach außen geschwenkt wird und damit außerhalb des Bewegungsbereichs des Anschlags 57 der Kupplungsscheibe 34a ist.

Aufgrund dieser Anordnung können die Schienenarme 1, 2 frei in Extensions- und Flexionsrichtung geschwenkt werden, wobei die Feder 22 eine entsprechende Vorspannkraft auf die Schienenarme 1, 2 in Extensionsrichtung aufbringt.

Figur 13b zeigt eine Stellung, in welcher die Federkraft von den noch verriegelten Schienenarmen 1, 2 abgekoppelt wird. Hierzu wird zunächst die Federkraftblockiereinrichtung aktiviert. Dies geschieht dadurch, dass die in Figur 1 dargestellte Handhabe 9 von der in Figur 1 gezeigten Freigabestellung in Richtung des Pfeils 70 manuell in eine Blockierstellung verschoben wird. Diese Verschiebebewegung verschiebt den Sperrschieber 59 (Figur 5) und damit das Federelement 61 entsprechend. Das Ende des Federelements 61 drückt damit den Verriegelungsabschnitt 56a der Sperrklinke 56 radial nach innen. Wird nun der erste Schienenarm 1 um etwa 45° in Flexionsrichtung bewegt, wird das Gehäuse 6 zusammen mit der Sperrklinke 56 entsprechend mitgedreht. Die Sperrklinke 56 gelangt dadurch in Verriegelungseingriff mit dem Anschlag 57. Die Schienenarme 1, 2 können sich nicht mehr in Extensionsrichtung bewegen. Die in Extensionsrichtung wirkende Federkraft ist damit blockiert.

Figur 13c zeigt, dass als nächster Schritt die Sperrriegel 10, 11 manuell derart auf den Schienenarmen 1, 2 verschoben werden, dass sie außer Eingriff mit den Rastausnehmungen 52a, 40 gelangen.

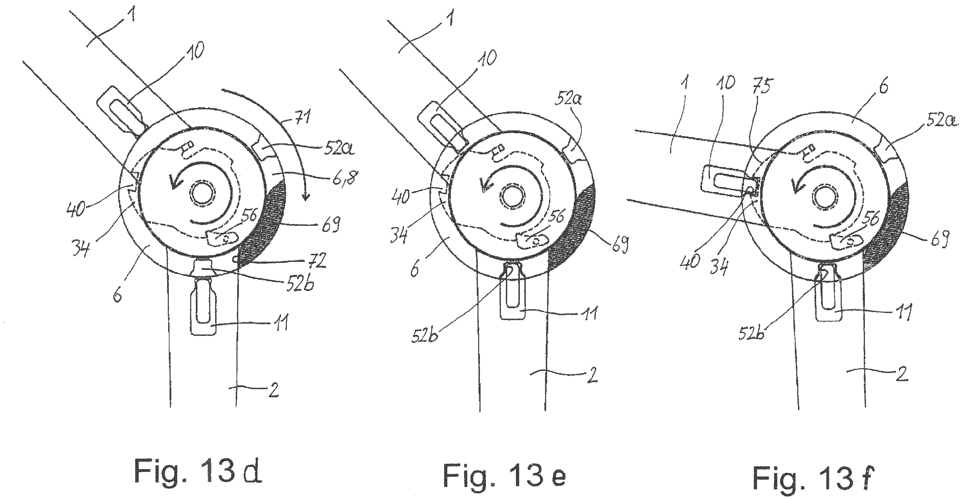

Figur 13d zeigt, dass anschließend das gesamte Gehäuse 6 relativ zu beiden Schienenarmen 1, 2 in Richtung des Pfeils 71 gedreht wird. Im gezeigten Ausführungsbeispiel beträgt der Drehwinkel etwa 100°. Die Drehung des Gehäuses 6 erfolgt soweit, bis die Rastausnehmung 52b dem zweiten Sperrriegel 11 gegenüberliegt, so dass der zweite Sperrriegel 11 in die Rastausnehmung 52b eingeführt werden kann. Zweckmäßigerweise schlägt in dieser Drehstellung eine Anschlagfläche 72 des erhabenen Abschnitts 69 am zweiten Schienenarm 2 an, so dass für den Benutzer die Drehendstellung des Gehäuses 6 sofort erkennbar ist. Bei der Drehbewegung des Gehäuses 6 wird das Verstellzahnrad 19, der Federträger 17, die Feder 22 und die Kupplungsscheibe 34a entsprechend mitgedreht.

Aus Figur 13e ist ersichtlich, dass die Sperrriegel 10, 11 aufgrund der in den Figuren 13a bis 13g nicht dargestellten Federn 73, 74 derart in Richtung Schwenkachse 5 zurückgeschoben worden sind, dass der Sperrriegel 11 in die Rastausnehmung 52b des Gehäuses 6 und der Sperrriegel 10 in einen seitlichen Freiraum des Gehäuses 6 zurückgeschoben werden. Nunmehr ist somit der zweite Schienenarm 2 mit dem Gehäuse 6 und damit mit dem inneren Federende 21 drehgekoppelt, während der erste Schienenarm 1 noch um einen bestimmten Winkelbereich um die Schwenkachse 5 schwenkbar ist.

Aus Figur 13f ist ersichtlich, dass im nächsten Schritt der erste Schienenarm 1 etwas weiter im Gegenuhrzeigersinn geschwenkt wird, bis der erste Sperrriegel 10 in die Rastausnehmung 40 der Kupplungsscheibe 34a einrastet. Hierbei gleitet der radial innere Endbereich des Sperrriegels 10 auf einer Anlaufschräge 75 der Kupplungsscheibe 34a. Der erste Schienenarm 1 ist damit mit der Kupplungsscheibe 34a und damit mit dem zweiten Federende 32 drehgekoppelt.

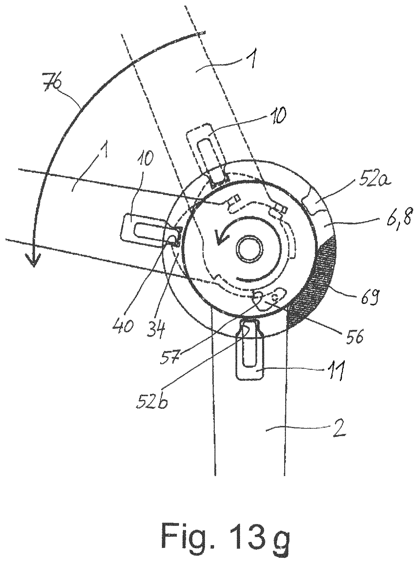

Aus Figur 13g ist ersichtlich, dass im nächsten Schritt die Federkraftblockiereinrichtung gelöst wird, damit die Feder 22 die Federkraft nunmehr, wie durch den Pfeil 76 angedeutet, in Flexionsrichtung auf die Schienenarme 1, 2 übertragen kann. Die Federkraftblockiereinrichtung wird dadurch gelöst, dass die in Figur 1 dargestellte Handhabe 9 manuell entgegen der Richtung des Pfeils 70 wieder in die in Figur 1 dargestellte Position zurückgeschoben wird. Hierdurch wird die Klinke 56 aus der Bewegungsbahn des Anschlags 57 herausgeschwenkt. Der Schienenarm 1 kann damit mittels der Feder 22 von der in Figur 13g gestrichelt dargestellten Stellung in Richtung der mit durchgezogenen Linien gezeigten Stellung geschwenkt werden. Die Schienenarme 1,2 des Orthesengelenks werden nun in Flexionsrichtung gedrängt.

## Patentansprüche

1. Dynamisches Orthesengelenk mit einem ersten Schienenarm (1) und einem zweiten Schienenarm (2), die in einem Gelenkabschnitt (3) gelenkig miteinander verbunden sind, wobei der Gelenkabschnitt (3) ein Gehäuse (6) und eine Feder (22) aufweist, welche die Schienenarme (1, 2) in Extensionsrichtung oder Flexionsrichtung drängt und ein erstes Federende (21) und ein zweites Federende (32) aufweist, wobei das erste Federende mit dem Gehäuse (6) drehgekoppelt ist und das zweite Federende (32) mit einer Federkraftübertragungseinrichtung (34) in Wirkverbindung steht,
**dadurch gekennzeichnet,**
**dass** das Gehäuse (6) relativ zu beiden Schienenarmen (1, 2) drehbar gelagert ist,
**dass** die mit dem zweiten Federende in Wirkverbindung stehende Federkraftübertragungseinrichtung (34) eine Kupplungsscheibe (34a) aufweist, die um eine Schwenkachse (5) drehbar ist,
**dass** das Orthesengelenk einen Umschaltmechanismus zum Umschalten zwischen einem ersten Betriebszustand, in dem die Federkraft die Schienenarme (1, 2) in Extensionsrichtung drängt, und einem zweiten Betriebszustand aufweist, in dem die Federkraft die Schienenarme (1, 2) in Flexionsrichtung drängt, wobei der Umschaltmechanismus eine zwischen den Schienenarmen (1, 2), dem Gehäuse (6) und der Federkraftübertragungseinrichtung (34) wirkende lösbare Drehkopplungseinrichtung umfasst, mit welcher der erste und zweite Schienenarm (1, 2) wahlweise mit dem Gehäuse (6) oder der Federkraftübertragungseinrichtung (34) in Drehkopplungsverbindung bringbar sind,
und **dass** das Gehäuse (6) bei gelöster Drehkopplungseinrichtung zwischen einer ersten Drehposition, in der das Gehäuse (6) mit dem ersten Schienenarm (1) drehfest verbindbar ist, während die Kupplungsscheibe (34a) mit dem zweiten Schienenarm (2) drehfest verbindbar ist, und einer zweiten Drehposition drehbar ist, in der das Gehäuse (6) mit dem zweiten Schienenarm (2) drehfest verbindbar ist, während die Kupplungsscheibe (34a) mit dem ersten Schienenarm (1) drehfest verbindbar ist.

2. Dynamisches Orthesengelenk nach Anspruch 1, **dadurch gekennzeichnet, dass** die Drehkopplungseinrichtung Rastausnehmungen (52a, 52b, 40), die am Gehäuse (6) und an der Kupplungsscheibe (34a) angeordnet sind, und Sperrriegel (10, 11) umfasst, die an den Schienenarmen (1, 2) festgelegt und zwischen einer in die Rastausnehmungen (52a, 52b, 40) eingreifenden Drehblockierstellung und einer aus den Rastausnehmungen (52a, 52b, 40) entfernten Drehfreigabestellung bewegbar sind.

3. Dynamisches Orthesengelenk nach Anspruch 2, **dadurch gekennzeichnet, dass** die Sperrriegel (10, 11) aus Schiebe- oder Schwenkriegeln bestehen, die verschiebbar oder schwenkbar an den Schienenarmen (1, 2) befestigt sind.

4. Dynamisches Orthesengelenk nach Anspruch 2 oder 3, **dadurch gekennzeichnet, dass** das Gehäuse (6) zwei Rastausnehmungen (52a, 52b) aufweist, die in Umfangsrichtung des Gehäuses um 100° bis 150° zueinander beabstandet angeordnet sind.

5. Dynamisches Orthesengelenk nach Anspruch 4, **dadurch gekennzeichnet, dass** das Gehäuse (6) ein napfförmiges Gehäusehauptteil (7) und ein axial über das Gehäusehauptteil (7) vorragendes, sich lediglich über einen Teil von dessen Umfang erstreckendes Gehäusesegment (8) aufweist, in dem mindestens eine der Rastausnehmungen (52a, 52b) vorgesehen ist.

6. Dynamisches Orthesengelenk nach einem der Ansprüche 2 bis 5, **dadurch gekennzeichnet, dass** mindestens einer der Sperrriegel (10, 11) mittels einer Feder (73, 74) in die Drehblockierstellung vorgespannt ist.

7. Dynamisches Orthesengelenk nach einem der Ansprüche 2 bis 6, **dadurch gekennzeichnet, dass** mindestens einer der Sperrriegel (10, 11) einen in der Drehblockierstellung selbsthemmenden Verriegelungskopf mit einer schräg zur Verschiebe- oder Schwenkrichtung der Sperrriegel (10, 11) angeordneten und mit einer Wand der Rastausnehmungen (52a, 52b, 40) in Anlage bringbaren Anlagefläche aufweist, welche die Wand hintergreift.

8. Dynamisches Orthesengelenk nach einem der vorhergehenden Ansprüche, **dadurch gekennzeichnet, dass** das Orthesengelenk eine Federkraftblockiereinrichtung zum Abkoppeln der Federkraft von den Schienenarmen (1, 2) aufweist.

9. Dynamisches Orthesengelenk nach Anspruch 8, **dadurch gekennzeichnet, dass** die Federkraftblockiereinrichtung ausgebildet ist, mit der Kupplungsscheibe (34a) der Federkraftübertragungseinrichtung (34) derart zusammenzuwirken, dass die Drehung der Kupplungsscheibe (34a) in einer Übertragungsrichtung der Federkraft wahlweise blockiert oder freigegeben wird.

10. Dynamisches Orthesengelenk nach Anspruch 9, **dadurch gekennzeichnet, dass** die Federkraftblockiereinrichtung einen an der Kupplungsscheibe (34a) angeordneten Anschlag (57) und eine Sperrklinke (56) aufweist, die mittels einer Handhabe (9) in die Bewegungsbahn des Anschlags (57) hineinbewegbar und aus dieser Bewegungsbahn entfernbar ist.

## Claims

1. Dynamic orthosis joint having a first rail arm (1) and a second rail arm (2), which are flexibly connected to each other in a joint section (3), wherein the joint section (3) has a housing (6) and a spring (22), which pushes the rail arms (1, 2) in the extension direction or flexion direction and has a first spring end (21) and a second spring end (32), wherein the first spring end is rotationally coupled to the housing (6), and the second spring end (32) is in active connection with a spring force transmission device (34),
**characterised in that**
the housing (6) is rotatably mounted in relation to the two rail arms (1, 2),
the spring force transmission device (34) in active connection with the second spring end has a coupling disc (34a), which can be rotated around a pivot axis (5),
the orthosis joint has a switching mechanism for switching between a first operating state, in which the spring force pushes the rail arms (1, 2) in the extension direction, and a second operating state, in which the spring force pushes the rail arms (1, 2) in the flexion direction, wherein the switching mechanism comprises a releasable rotational coupling device acting between the rail arms (1, 2), the housing (6) and the spring force transmission device (34), with which rotation coupling device the first and second rail arm (1, 2) can selectively be brought into rotational coupling connection with the housing (6) or the spring force transmission device (34),
and, when the rotational coupling device is released, the housing (6) can be rotated between a first rotational position, in which the housing (6) can be non-rotationally connected to the first rail arm (1), while the coupling disc (34a) can be non-rotationally connected to the second rail arm (2), and a second rotational position, in which the housing (6) can be non-rotationally connected to the second rail arm (2), while the coupling disc (34a) can be non-rotationally connected to the first rail arm (1).

2. Dynamic orthosis joint according to claim 1, **characterised in that** the rotational coupling device comprises latching recesses (52a, 52b, 40), which are arranged on the housing (6) and on the coupling disc (34a), and locking bolts (10, 11), which are fixed to the rail arms (1, 2) and can be moved between a rotational blocking position engaging in the latching recesses (52a, 52b, 40) and a rotational release position removed from the latching recesses (52a, 52b, 40).

3. Dynamic orthosis joint according to claim 2, **characterised in that** the locking bolts (10, 11) consist of shifting or pivoting bolts, which are shiftably or pivotably fixed on the rail arms (1,2).

4. Dynamic orthosis joint according to claim 2 or 3, **characterised in that** the housing (6) has two latching recesses (52a, 52b) which are arranged spaced apart from each other by 100° to 150° in the peripheral direction of the housing.

5. Dynamic orthosis joint according to claim 4, **characterised in that** the housing (6) has a bowl-shaped housing main part (7) and a housing segment (8) which protrudes axially beyond the housing main part (7) and only extends across a part of its periphery and in which at least one of the latching recesses (52a, 52b) is provided.

6. Dynamic orthosis joint according to one of claims 2 to 5, **characterised in that** at least one of the locking bolts (10, 11) is pretensioned into the rotational blocking position by means of a spring (73, 74).

7. Dynamic orthosis joint according to one of claims 2 to 6, **characterised in that** at least one of the locking bolts (10, 11) has a locking head, which is self-retaining in the rotational blocking position, having an abutment surface arranged obliquely in relation to the shifting or pivoting direction of the locking bolts (10, 11) and able to be brought into abutment with a wall of the latching recesses (52a, 52b, 40), said abutment surface engaging behind the wall.

8. Dynamic orthosis joint according to one of the preceding claims, **characterised in that** the orthosis joint has a spring force blocking device for decoupling the spring force from the rail arms (1, 2).

9. Dynamic orthosis joint according to claim 8, **characterised in that** the spring force blocking device is formed to interact with the coupling disc (34a) of the spring force transmission device (34) in such a way that the rotation of the coupling disc (34a) in a transfer direction of the spring force is selectively blocked or released.

10. Dynamic orthosis joint according to claim 9, **characterised in that** the spring force blocking device has a stop (57) arranged on the coupling disc (34a) and a pawl (56), which can be moved into the movement path of the stop (57) by means of a handle (9) and can be removed from this movement path.

## Revendications

1. Articulation dynamique d'orthèse comprenant un premier bras d'attelle (1) et un deuxième bras d'attelle (2) reliés articulés l'un à l'autre dans une partie d'articulation (3), ladite partie d'articulation (3) présentant un boîtier (6) ainsi qu'un ressort (22) qui sollicite les bras d'attelle (1, 2) dans le sens de l'extension ou de la flexion et qui présente une première extrémité de ressort (21) et une deuxième extrémité de ressort (32), ladite première extrémité de ressort étant couplée rotative au boîtier (6) et la deuxième extrémité de ressort (32) étant en liaison fonctionnelle avec un dispositif de transmission de force de ressort (34),
**caractérisée en ce que**
le boîtier (6) est monté rotatif par rapport aux deux bras d'attelle (1, 2),
le dispositif de transmission de force de ressort (34) qui est en liaison fonctionnelle avec la deuxième extrémité de ressort présente une plaquette d'embrayage (34a) susceptible de tourner sur un axe de pivotement (5),
l'articulation d'orthèse présente un mécanisme de sélection permettant de passer d'un premier état fonctionnel, dans lequel la force de ressort sollicite les bras d'attelle (1, 2) dans le sens de l'extension, à un deuxième état fonctionnel, dans lequel la force de ressort sollicite les bras d'attelle (1, 2) dans le sens de flexion, et vice versa, ledit mécanisme de sélection comprenant un dispositif de couplage en rotation débrayable agissant entre les bras d'attelle (1, 2), le boîtier (6) et le dispositif de transmission de force de ressort (34) et avec lequel les premier et deuxième bras d'attelle (1, 2) peuvent être sélectivement couplés en rotation avec le boîtier (6) ou le dispositif de transmission de force de ressort (34), et
en position débrayée du dispositif de couplage en rotation, le boîtier (6) peut tourner entre une première position de rotation, dans laquelle ledit boîtier (6) peut être solidarisé en rotation avec le premier bras d'attelle (1), tandis que la plaquette d'embrayage (34a) peut être solidarisée en rotation avec le deuxième bras d'attelle (2), et une deuxième position de rotation, dans laquelle ledit boîtier (6) peut être solidarisé en rotation avec le deuxième bras d'attelle (2), tandis que la plaquette d'embrayage (34a) peut être solidarisée en rotation avec le premier bras d'attelle (1).

2. Articulation dynamique d'orthèse selon la revendication 1, **caractérisée en ce que** le dispositif de couplage en rotation comprend des crans (52a, 52b, 40) disposés au niveau du boîtier (6) et de la plaquette d'embrayage (34a), ainsi que des clavettes de verrouillage (10, 11) fixées aux bras d'attelle (1, 2) et mobiles entre une position de blocage en rotation par emboîtement dans les crans (52a, 52b, 40) et une position de libération en rotation par dégagement hors des crans (52a, 52b, 40).

3. Articulation dynamique d'orthèse selon la revendication 2, **caractérisée en ce que** les clavettes de verrouillage (10, 11) se composent de clavettes coulissantes ou pivotantes qui sont fixée aux bras d'attelle (1, 2) de manière à pouvoir coulisser ou pivoter.

4. Articulation dynamique d'orthèse selon la revendication 2 ou 3, **caractérisée en ce que** le boîtier (6) présente deux crans (52a, 52b) disposés dans le sens de la périphérie du boîtier selon un écart de 100° à 150°.

5. Articulation dynamique d'orthèse selon la revendication 4, **caractérisée en ce que** le boîtier (6) présente une partie principale de boîtier (7) en forme de cupule et un segment de boîtier (8) faisant axialement saillie au-dessus de la partie principale de boîtier (7) et s'entendant uniquement sur une partie de la périphérie de cette dernière, dans lequel est prévu au moins un des crans (52a, 52b).

6. Articulation dynamique d'orthèse selon l'une des revendications 2 à 5, **caractérisée en ce qu'**au moins une des clavettes de verrouillage (10, 11) est prétendue, au moyen d'un ressort (73, 74), dans la position de blocage en rotation.

7. Articulation dynamique d'orthèse selon l'une des revendications 2 à 6, **caractérisée en ce qu'**au moins une des clavettes de verrouillage (10, 11) présente une tête de verrouillage autobloquante dans la position de blocage en rotation, laquelle comporte une surface de butée oblique par rapport au sens de coulissement ou de pivotement des clavettes de verrouillage (10, 11) et susceptible de venir en butée, par l'arrière, avec une paroi des crans (52a, 52b, 40).

8. Articulation dynamique d'orthèse selon l'une quelconque des revendications précédentes, **caractérisée en ce que** l'articulation d'orthèse présente un dispositif de blocage de force de ressort destiné au découplage de la force de ressort par rapport aux bras d'attelle (1, 2).

9. Articulation dynamique d'orthèse selon la revendication 8, **caractérisée en ce que** le dispositif de blocage de force de ressort est conçu pour coopérer avec la plaquette d'embrayage (34a) du dispositif de transmission de force de ressort (34) de manière à bloquer ou à permettre sélectivement la rotation de la plaquette d'embrayage (34a) dans un sens de transmission de la force de ressort.

10. Articulation dynamique d'orthèse selon la revendication 9, **caractérisée en ce que** le dispositif de blocage de force de ressort présente une butée (57) disposée au niveau de la plaquette d'embrayage (34a) et un cliquet (56) qui, au moyen d'une poignée (9), est susceptible d'être placé dans la trajectoire de la butée (57) et éloigné de cette trajectoire.
